(19) 

Europäisches
Patentamt

European
Patent Office

Office européen
des brevets

(11) **EP 4 654 203 A1**

(12) **EUROPEAN PATENT APPLICATION**

(43) Date of publication:
 26.11.2025 Bulletin 2025/48

(51) International Patent Classification (IPC):
 ***G16B 5/00*** *(2019.01)* ***G16B 20/00*** *(2019.01)*
 ***G16B 40/20*** *(2019.01)*

(21) Application number: 24177290.4

(22) Date of filing: **22.05.2024**

(52) Cooperative Patent Classification (CPC):
 **G16B 5/00; G16B 20/00; G16B 40/20**

(84) Designated Contracting States:
 **AL AT BE BG CH CY CZ DE DK EE ES FI FR GB
 GR HR HU IE IS IT LI LT LU LV MC ME MK MT NL
 NO PL PT RO RS SE SI SK SM TR**
 Designated Extension States:
 **BA**
 Designated Validation States:
 **GE KH MA MD TN**

(71) Applicant: **Camino Science Sp. z o.o.**
 **15101 Bialystok (PL)**

(72) Inventors:
 • **Ferenc, Mariusz**
 **07-320 Malkinia Gorna (PL)**
 • **Pietrzycki, Marcin**
 **15-674 Bialystok (PL)**
 • **Sawulski, Bartlomiej**
 **16-010 Nowodworce (PL)**

(74) Representative: **Jedrzejewski, Michal**
 **KJP Kurcman Jedrzejewski i Partnerzy
 ul. Klobucka 25A/61
 02-699 Warszawa (PL)**

(54) **COMPUTER-AIDED METHOD FOR MODELING PHAGE-HOST INTERACTIONS**

(57) A computer-aided method for modelling phage-host interactions using deep-architecture neural networks and machine learning, according to the invention, is characterised in that it is carried out in the following steps:
Stage I - application of the directional model;
Stage II - application of models of a specific group of defence and anti-defence systems.

EP 4 654 203 A1

## Description

The object of the invention is a computer-aided method for modelling phage-host interactions.

The evolution of bacteria is shaped by phages. Interactions between bacteria and phages lead bacteria to produce specific molecular tools in the form of defence systems. This has initiated the evolution of phage anti-defence systems, potentially neutralising defence systems. This is, in practice, a continuous arms race leading to the evolution of defence (antiviral) and anti-defence (antimicrobial) mechanisms.

Reliable prediction of phage-host interactions is a critical challenge for understanding the molecular mechanisms of this interaction and has applications in important areas of biology, from phage therapy to gene editing using the CRISPR-Cas defence system to food production (such as fermented dairy products). At the same time, a reliable method for modelling phage-host interactions makes it possible to track the contribution of specific genes or their syntenic systems and their synergies/antagonisms in phenotypes: infection or immunity. Specifically here, these are understood as syntenic systems that form defence systems (with a bacterial defence function against phages) [10] and phage anti-defence systems (that inactivate a given bacterial defence system and enable successful infection) [15]. On top of this, an important element incorporated into the mechanism for predicting phage-host interactions is the building of a full bacterial immune barrier through so-called pan-immunity [10]. This means that, although a single strain cannot carry all possible immune defences (due to fitness burden), it can use the mechanism of Horizontal Gene Transfer(HGT), allowing it to access the immune defence mechanisms held in its repertoire by closely related strains. Thus, the immune system is formed by several closely related organisms (its pangenome) instead of the genome of a single microorganism. In practice, therefore, the immune system of a given bacterial host comprises the sum of all the defences available to a given microorganism via HGT from its arsenal of closely related microorganisms for its own use.

Below is a description of a machine learning method based on an artificial intelligence modelling approach for detecting novel virus-host interactions, both direct and indirect. Our method embeds the protein sequence of each gene in both a genomic and evolutionary context. This embedding allows us to identify: (1) existing interactions that are not included in the training set, (2) previously uncharacterised interactions and (3) interactions from classes/families absent from the training set.

The thought vector of the experiments conducted in the area of AI / ML modelling development was to create universal methodologies for encoding molecular data. In practice, this was to address the following objectives:

  a. Enabling the highest possible quality of generalisation properties of models oriented towards a specific type of polygenic patterns (gene syntenes as in the case of defence systems) or their groups forming synergies / antagonisms,

  b. the possibility to carry out a full-fledged Discussion of the Phenotype Pattern: infectivity / resistance in the case of corresponding functional spaces centred on a given molecular pattern (single defence system or co-occurring defence systems). Such a discussion results in the extraction of factors (genes / gene systems and their co-occurrence patterns) responsible for the functioning of a given pattern and their variability affecting the value of the inferential/resistance phenotype pattern function,

  c. automatic scaling of the solution, which allows a given user to build and navigate through thousands of models oriented towards different constellations of defence systems.

State of the art

Although the scientific world has already managed to discover several hundred defence systems, there are still few known systems that modulate them, which are called anti-defence systems. At present, these are mainly anti-CRISPR (acr) systems and a few less numerous ones that target other newly identified defence systems (e.g. anti-Thoeris, anti-Gabija [16]). This is due to several factors - mainly the fact that the mechanisms of action of bacterial defence systems counteracting viral infection are very diverse in terms of syntenic and protein structure and, at the same time, the very nature of the mechanism of action. This can be seen, among other things, in the proteins produced by anti-CRISPR (acr) genes. At the same time, these are usually small proteins (up to 200 amino acids), which makes it even more difficult to identify their functional properties (their domains including those involved in binding affinity). Methods that have emerged to date for the detection of anti-defence systems are usually based on classical methods that encode the properties of already known proteins - based on sequence homology (e.g. using Hidden Markov Models ( HMM)) [13]. In some cases, this is effective in practice, but given the high diversity of these proteins, finding new case types that are homologously different from those already known is very limited in terms of efficiency. The second type of methodology is to look for Interactions: Protein-Protein (between a given protein on the side of the virus and specific proteins on the side of the defence system of a given bacterial host). This method, however, is extremely computationally greedy (due to the number of combinations of possible protein-protein interaction pairs and their verification times). So far, no universal and efficient method for their detection has been proposed.

The above problems have been solved by the method which is the subject of the present invention.

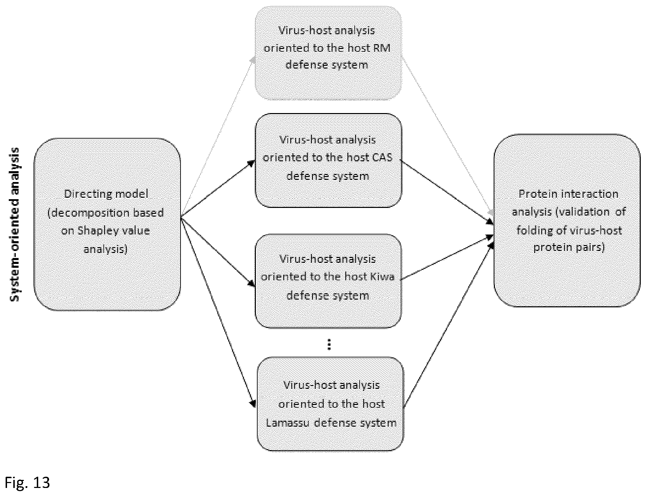

The computer-assisted method for modelling phage-host interactions using deep-architecture neural networks and machine learning, according to the invention, is characterised by the fact that it is carried out in two classification stages (Fig.13). The first stage (directional model) is an initial model that, for a given genome pair: phage-bacteria, determines which defence/anti-defence systems are relevant to the infection/resistance phenotype. After this narrowing down, the method proceeds to step two, where, for a given phage-bacteria pair, a more precise classification takes place using a model or models focused on a given defence system or a close group of such systems ( being in synergy). The sequences of steps for both stages are characterised below:

Stage I - Directional model

The aim of this method is to reconstruct the bacteriophage-bacterial host interaction based on the encoding at the input to this model of information about the presence in a given pair: the bacterium and the potentially infecting bacteriophage of genes from, respectively:

a. the fullest possible collection of defence systems on the part of the bacterial host
b. collection, as complete as possible, of anti-protective systems on the bacteriophage side

This in practice makes it possible, on the one hand, to capture the entire defence / anti-defence mechanism due to its pan-immune nature (built as a superposition of several or more defence systems and their mutual synergies / antagonisms). On the other hand, the method allows narrowing down in the model thus built already for individual pairs( bacteriophage/bacterial host): which specifically of the defence/anti-defence systems are activated at the core of the resistance/infection mechanism. In this way, the method allows the prediction to be redirected to a more specific type of coding already focused on individual defence systems and, very importantly: to observe the specific modulators of the respective defence system created by the bacteriophage ( Stage II of the method).

The sequence of subsequent activities performed in Stage I is listed below:

1) identification of known defence systems on the host genome side (checking for the presence of gene syntenes of a given defence system from a group of at least 91 candidate defence systems);
2) identification of known anti-defence systems on the genome side of the phage using an indicator classifier (checking for the presence of genes from a group of at least 20 candidate anti-defence systems, with cases with a probability of belonging to a class above 0.9 being selected);
3) The presence of individual gene groups within the virus and host is noted in the model input matrix through 3 values in the range (0 - 1):

- At the intersection of the two systems present: host defence and anti-defence phage: a value of: 1.0 is set.
- The presence of a single host defence system or phage anti-system is represented by the value: 0.5.
- The remaining fields are filled in by a value of 0.0, with this type of representation taking into account all combinations of known defence vs. anti-defence systems without initially directing the model analysis to a selected group of genes, e.g. from a system/anti-system gene context(this is only in Stage II);

4) The data representation described above then serves as input to a classification model using two-dimensional convolution layers on the aforementioned coding matrix, whereby: the aim of the network is to assess the probability of phage-host interaction as efficiently as possible based on the co-occurrence matrix of the bacterial systems.
5) The convolutional network created in the next step is subjected to neural activation analysis using an additive feature assignment approach based on Shapley values [11] [12], where the highest values in the input matrix indicate a group of defence/anti-defence systems that are important in the classifier's decision-making process, whereby, the systems determined through this step: defence/anti-defence systems are used in stage II of the construction of classification models targeting detailed information about the aforementioned systems;

Stage II - Targeted models of a specific group of defence and anti-defence systems:

1) identification of host-side defence systems indicated by stage I (reference genes/proteins);
2) identification of the bacteriophage-side systems (reference genes/proteins) indicated by step I;
3) each of the above-identified genes producing reference (bacteriophage/host) proteins, are encoded in a form that expresses both:

a. protein properties for a reference set of genes (gene synteny),
b. properties of proteins produced by the context of neighbouring genes relative to the reference gene synteny,
c. The coding of the above proteins is in the form of dense vectors and is carried out as follows: for a selected reference gene-synteny, the context of the neighbouring genes (n genes upstream and n genes downstream of the gene-synteny) is established, whereby all proteins formed from sequences of key genes (central, belonging to the synteny of a given defence system) and neighbouring genes are coded using dense vectors of dimension d = 128. Such vectors representing the individual aforementioned proteins are then combined, preserving the order of occurrence of the genes to which they relate, into a combined form into a single tensor. The tensors thus created, reflecting the topology of the genes comprising the synteny and its context, further allow complex types of syntenic patterns to be established through a Convolutional Neural Network (CNN) master model operation;

4) Encoding the phage taxonomy to capture pan-immune patterns into dense vector form;

5) encoding the host taxonomy to capture pan-immune patterns into a dense vector form;

6) concatenation of all the above encoded dense vectors in the form of an input tensor;

7) Training an AI/ML (Convolutional Neural Network) model of phage-host interaction using a dataset of pairs: phage-host.

8) on the above-trained model for a given input tensor of a phage-host pair, the identification of which of its features are most relevant for decision-making (with the help of an additive feature assignment approach based on Shapley values [11][12]) by the neural network for: infection/resistance phenotypes, with these traits mapped to proteins encoded in the phage-host tensor.

The process of extracting the most relevant proteins in a phage-host tensor involves determining the Shapley values for the entire tensor. Knowing that each successive 128 features in the tensor represents a specific bacterial or bacteriophage protein, we are able to identify which Shapley values, and therefore: which proteins are most relevant to the classifier's decision about host infection or immunity against a particular virus. The analysis assumed that for each range of 128 features, a representative of the section was identified as the maximum, mean or median value from the range. The full array of proteins in the tensor was then globally analysed considering both bacterial and viral proteins. Combinations of these proteins allow a detailed determination of which features are potentially most relevant.

The method according to the invention allows a more versatile approach to the detection of the proteins mentioned, by approaching the problem from a different perspective - namely from the side of modelling the properties of the interaction between the phage and its bacterial host. Starting from the fact that this interaction is determined by or linked to specific genes or gene systems (including, in particular, infection or resistance genes), it is possible to identify the relevance of the genes/gene systems in question and to further search for the other genes determining it. Hence, starting from the phage-host interaction - having it reconstructed by means of a convolutional network model and transferring the relevant features of the molecular patterns in its internal representation (a. Protein properties and similarities for genes / gene syntenic systems and b. Contexts of neighbouring genes (containing gene syntenic systems)), it is possible to observe phage inhibitors of given bacterial host defence systems as a derivative of the phage-host interaction.

In summary - in this approach, starting from a method of reliable interaction prediction and appropriate construction of the input tensors and further analysis of the activation of the individual genes encoded therein (on the viral and bacterial side, respectively) - the factors behind a given prediction of infection and immunity phenotype can be effectively found.

Examples of the implementation elements of the invention are shown in the individual drawings in which:

Fig. 1 shows the representation of the data in the directional model of the Fag-Host interaction;

Fig. 2 shows the architecture of a Convolutional Neural Network for predicting Fag-Host interactions in a directional model;

Fig. 3 shows a view of the input tensor with the shades (activation levels) of the given features and the columns and rows in the tensor indicating the defensive and anti-defensive systems, respectively, which are the basis for the creation of targeted classifiers in stage II;

Fig. 4 shows a diagram of the data flow and conversion in the prediction engine;

Fig. 5 shows the input tensor diagram for a given phage-host pair, which is the input for the classification engine;

Fig. 6 shows the main model of the Fag-Host interaction architecture;

Fig. 7 shows a general schematic of the indicator model of the anti-defence proteins present on the bacteriophage side;

Fig. 8 shows the architecture of the multilayer perceptron (MLP) indicator model of bacteriophage;

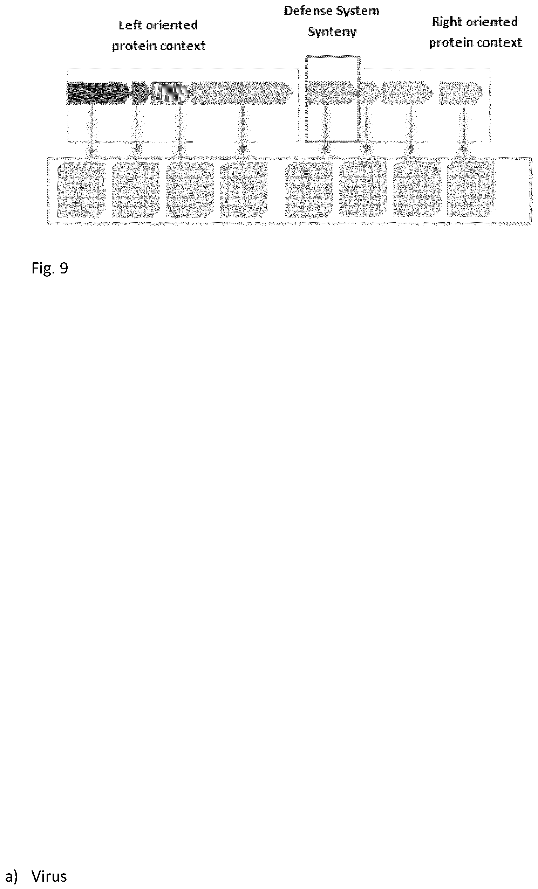

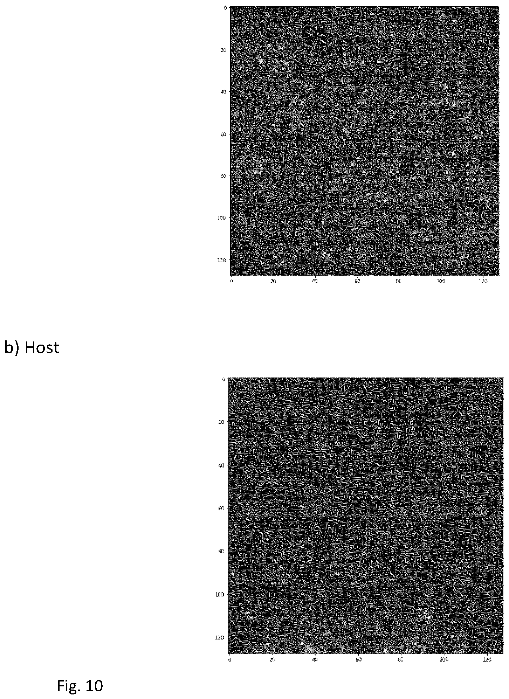

Fig. 9 shows the gene/protein contextual coding scheme;

Fig. 10 shows an example of a graphical fractal representation using the FCGR method for the genome examples (a) phage ( NC_028772.1), (b) bacterial host (GCA_001054315);

Fig. 11 shows the architecture of a convolutional neural network for a taxonomic phage classifier;

Fig. 12 shows the architecture of a convolutional neural network for a bacterial taxonomic model;

Fig. 13 Schematic of the use of a two-stage analysis using a Stage I directional model and systems-oriented models within the phage-host interaction as described for Stage II;

Fig. 14. shows the architecture of the Auto-Encoder model, which allows the dimension of the vectors produced by ProtBert to be reduced from d=1024 to d=128.

1. Definition of the body

The corpus consists of a collection of phage-host pairs, which are ordered pairs of genomes: *(m, n)*. To make it possible to carry out supervised learning, all instances of pairs in the corpus have class labels. Thus, the definition of a corpus is: $L_n = \{x_i, y_i\}_{i=1}^n$ , where each sample is described as a pattern: $x_i \ \varepsilon X$, corresponding to a pair of genomes$(m_i, n_i)$ with an attached expert response (class label) $y_i \in \Omega = \{\omega_1, ..., \omega_K\}$.

The required genome data ( individually for: phages, bacteria ) should meet the requirements of the fasta format, which describes nucleotide sequences. It is applied to the data corpus: training, testing and validation, as well as to the phage and host genomes provided by the solution user.

All genomes were retrieved from specific publicly available sources, including: NCBI (ncbi.nlm.nih.gov), NCBI Virus, Virus-Host DB, RefSeq, UniProt, MVP and IntAct. There were n = 7707 DNA sequences on the bacterial side and n = 2003 DNA sequences on the phage side.

2. Methodology

Stage I Fag-Host interaction model in a systems combination approach (directional model)

The first step of Stage I of the phage-host relationship study consists of preparing a representation to determine the occurrence of specific protein groups in both types of genomes (phage and bacterial) on a given data corpus.

In order to be able to perform efficient coding of information on the co-occurrence in a given phage-host pair of anti-systems and defence systems, a clustering analysis must be performed to establish identical sets of gene syntenes. In practice, we establish a set of common clusters on the basis of the defence system protein tags extracted using PADLOC [5] and DefenseFinder[6] software. For a corpus defined as above, at least 91 bacterial systems remain within consideration. In order to prepare a set of anti-defence systems in bacteriophage genomes, an indicator model was created to identify the locations in the genome of genes producing anti-defence proteins. On the aforementioned corpus, grouping these proteins yields 20 families of anti-defence proteins.

Indicator model for the identification of anti-defence proteins

Based on the scientific literature, a set of protein data has been compiled on the anti-CRISPR mechanisms discovered so far. Among these, anti-CRISPR (ACR) proteins are a large part. They are special because, as the scientific literature shows, other anti-defence genes may be located close to ACR genes. At the same time, some of the ACR proteins may additionally be inhibitors of other defence systems than CRISPR-Cas. The preparation of a model to carry out the determination of proteins important in the function of the bacterial anti-system was carried out starting from the data collection process by extracting proteins coinciding with those available in the literature (dbAPIS[8]). This process of comparing the similarity of the dense vectors (generated through ProtBert, each with dimension d=1024) of the above-mentioned protein templates with the proteins available in the IMGVR database [9], representing individual viral protein sequences, allowed the extraction of 20 groups of characteristic bacteriophage proteins that perform the aforementioned anti-system function.

In order to prepare the classification model, the above set of anti-defence protein sequences should be encoded by ProtBert Mean Encoder into a dense vector (dimensionality: d=1024). By the term ProtBert Mean Encoderwe mean here the procedure for averaging the vectors generated by ProtBert. To be precise: the procedure takes as input the vectors obtained from ProtBert (d=1024) that were generated for each of the amino acids comprising the protein in question and then calculates the arithmetic mean of these vectors.

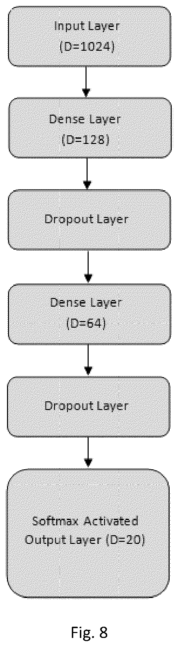

In the next step, having this input form, a multilayer perceptron (MLP) classification model with architecture as in Fig. 8 was prepared in a cross-validation approach.

Input data representation and architecture of the directional model

To carry out the analysis of each case of phage-host genome pairs, it is therefore necessary to identify the defence systems on the bacterial side and the candidates of the anti-defence systems having an indicator model prepared as above.

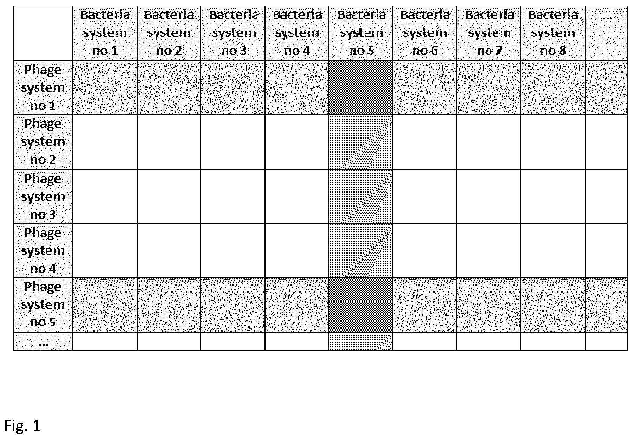

A second-order tensor is created to encode information about the systems identified as above. In such a tensor, the rows correspond to individual groups of bacteriophage anti-defence systems, while the columns represent individual bacterial systems. The dimensions of the tensor are thus: k x l, respectively, where: k - the number of types of defence systems and l - the number of types of anti-defence systems (occurring on the data corpus). An example of how the data are represented is shown in Fig. 1.

In Fig. 1, the light grey colour indicates in the matrix the occurrence of a specific anti-defence system in the bacteriophage genome (e.g. as in row No 1). The grey colour as in the cells of column No 5 symbolises the occurrence of a specific defence system in the bacteriophage genome. The dark grey colour (as in the beginning/end of column No 5) symbolises the intersection of the above-mentioned cases, which indicates the simultaneous occurrence of specific defence and anti-defence systems in the bacteriophage and bacterial genomes respectively.

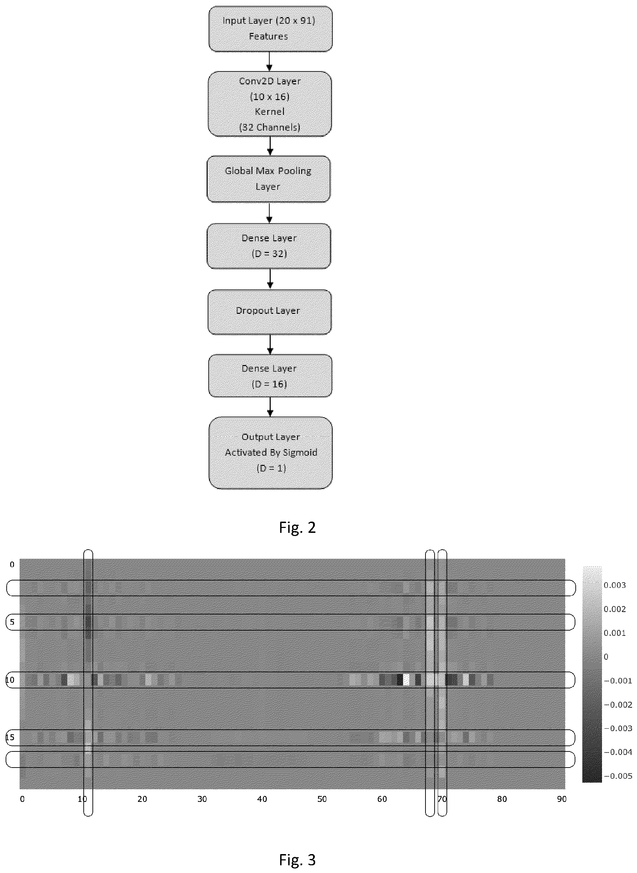

The tensor representation defined above is the input to the classifier model handling the determination of the virus-host interaction probability level(directional model). The model architecture is shown in Fig. 2. The classifier should be trained in a cross-validation approach.

The classifier model prepared in the approach defined as above allows an analysis by tracing the leading factors (genes, gene systems and their constellations) in phage-host interaction decisions. Based on the decomposition of system relevance, it can be determined which combinations of anti-defence and defence systems can be used for further analysis in a targeted modelling approach. By analysing neuronal activation using an additive feature assignment approach based on Shapley values [11][12], the relevance values of individual cells in the input tensor of the above model are first determined. Having these calculated, the significance values of the data are then calculated: systems / anti-systems. These are defined as: the sum of the changes in the significance of the individual cells in the column / row (corresponding to the activation of a given respectively: system / anti-system defence ) occurring in the input tensor of the Stage I classification mechanism. We write them with the following formula:

$$V_{active} = \sum_{i=0}^{i=N-1} {\textstyle\square}\, |x_{i+1} - x_i|$$

Equation 1

Fig. 3 shows the activations extracted by the pattern defined above using the example of the pair: bacterium-phage named: GCF_001856165.1 (bacterial host), NC_008364 (phage). The columns marked with a rectangular shape represent the defence systems activated for the pair as important in the bacterium's construction of resistance against the virus. At the same time, the rows extracted as active and marked with a rectangular shape are the relevant defence systems. Both pieces of information form the basis for building models targeting active defence and anti-defence systems that are built in step II.

Stage II Targeted models of a specific group of defence and anti-defence systems

The main deep learning model predicts the resistance or susceptibility of bacteria to a given phage infection. At the same time, and crucially: the way the model is set up allows identification:

    a. Bacterial gene syntenes are responsible for the ability of the bacterial host to defend itself against the phage,
    b. co-activations(synergies) between such defences that form a common line of defence,
    c. virus-side genes produce proteins that inactivate or antagonise specific host-side defences or that antagonise other viruses.

The concept of the method according to the invention described here starts from the problem of the interaction between a phage and its host. In particular, it concerns the modelling of the ability of a given phage to infect its host and the potential immunity of the host. Having a dataset of phage-host interaction pairs (m,n) and attached information on whether the virus infects a given bacterial host or whether the bacterium successfully defends itself (obtained from validated in vitro/in vivo tests in the laboratory), allows the creation of an appropriate artificial intelligence/machine learning model. In practice, the model predicts the aforementioned infectivity/resistance capabilities of the phage and the bacterial host, respectively. At the same time, the approach creates individual models targeting the particular defence system targeted by the model described in Stage I. The individual models are focused on a given defence system (or group of systems exhibiting synergistic action) - they model multi-layered topologies that reconstruct the geometry of phage-host interactions for a given defence system in the generated function space. By moving along specific trajectories on such a topology, it is possible to observe what are the factors (genes, gene syntenes) contributing to infection/immunity and their interactions (including Protein-Protein Interactions). This also allows the observation of pan-immune clusters.

The selection of specific individual models oriented towards a specific subset of defence systems is done as a result of the directional model described in Stage I.

Methodology stream: Stage I, constructing and running a directional model, is a preparation for Stage II, where more specific models (focused on a small subset: one or more defence systems - each with individual gene contexts on the phage side) are constructed and run. The specific deep learning tensors representing the encoded phage-host pairs in Stage I are constructed (according to Fig. 1 ), and the convolutional neural network model performs a convolution on the given input to identify pairs of mutually correlated anti-defence and defence systems (model architecture in Fig. 2). In the next step, it decomposes the set of defensive systems present in the so-called bacterial defence islands into a subset of the most relevant systems according to the methodology described above ( using Formula No. 1 and the diagram in Fig. 3). The defence systems/anti-defences thus extracted in Step I are the basis for the creation of a set of specific individual models in Step II (each oriented to and based on particular defence systems: reconstructing specific functions underlying resistance to viral infections and the mechanisms that inactivate or antagonise them on the phage side).

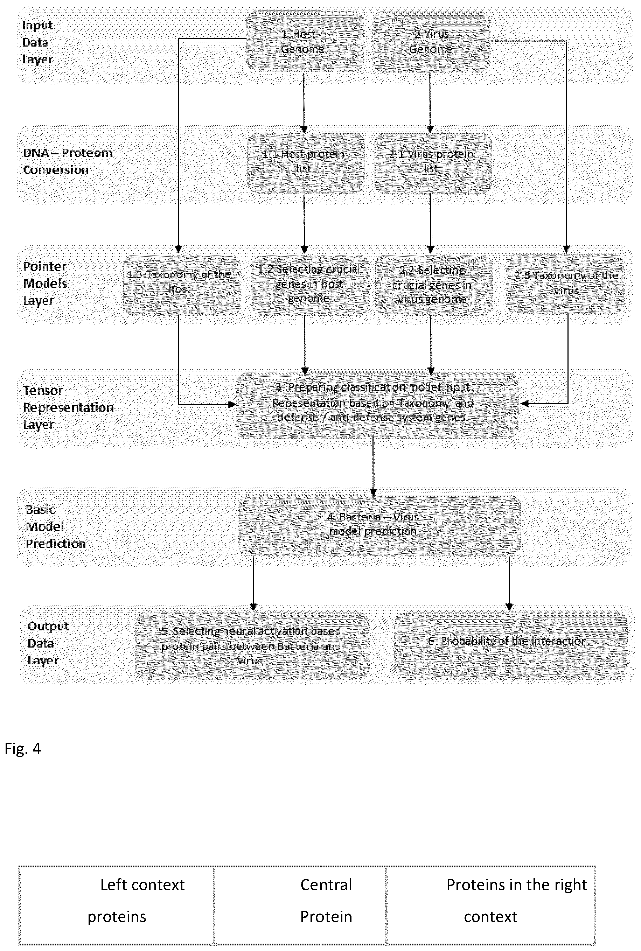

Fig. 4 shows the main processing scheme of the AI / ML approach methodology for predicting virus-host interactions. At the same time, this particular approach focuses on bacterial defence islands (containing a subset of defence systems) and potential regions of anti-defence systems on the phage side. In practice, this allows the phage-host interaction model to separate the interaction patterns of defence and anti-defence systems. At the same time, it allowed the identification of correlations between specific anti-defensive and defensive systems.

The input to the system (steps: 1 and 2) consists of the two genomes of the phage in question and its bacterial host, respectively. The genome of the host and the phage is represented by finite sequences $(a_i)_{i=1}^n, (b_j)_{j=1}^m$ elements $a_i, b_j \in \{A, C, T, G\}$. These sequences are then mapped to finite sequences of . $(A_i)_{i=1}^N, (B_j)_{j=1}^M$ elements of a 20-element set $\{A, C, D, E, F, G, H, I, K, L, M, N, P, Q, R, S, T, V, W, Y\}$ where each element of this set represents an amino acid. These amino acid sequences describe subsequent proteins. The mapping process is described in section 2.2.1 below. Each proteomic sequence is then transformed by a model from the BERT (encoder) family. In particular, ProtBert[1] was used here. It was initially trained on the Uniref100[3] corpus, a dataset of 217 million protein sequences. An implementation of ProtBert's Hugging Face [2] was used here [1], which allows protein sequences to be encoded in dense vectors (each of dimension d=1024). Such a representation yields a vector space metric, providing a metric for protein similarity in models seeking complex patterns based on combinations of specific protein families. In the case of the method presented here, such vectors embedded in the aforementioned vector space were used to encode individual proteins produced by specific genes of the phage-host pair, considered by the system to be potentially crucial for phage-host interactions. Specifically, inputs to initial models that help decide whether a particular protein is likely to belong to a group considered crucial for further processing of phage-host interaction models were encoded in this way. In addition, to reduce dimensionality, dedicated models were built independently for the phage and the bacterial host, which focus on specific types of proteins on both sides. This type of approach reduces the aforementioned dense vectors (with dimensionality d=1024) to d=128 and is described in section 2.2.2.

Since the taxonomic assignment of the phage and bacterial host determines their interaction (including taxonomic pan-genomic connotations), two classifiers have been developed that produce dense vectors representing taxonomic assignments, individually for the viruses and their hosts.

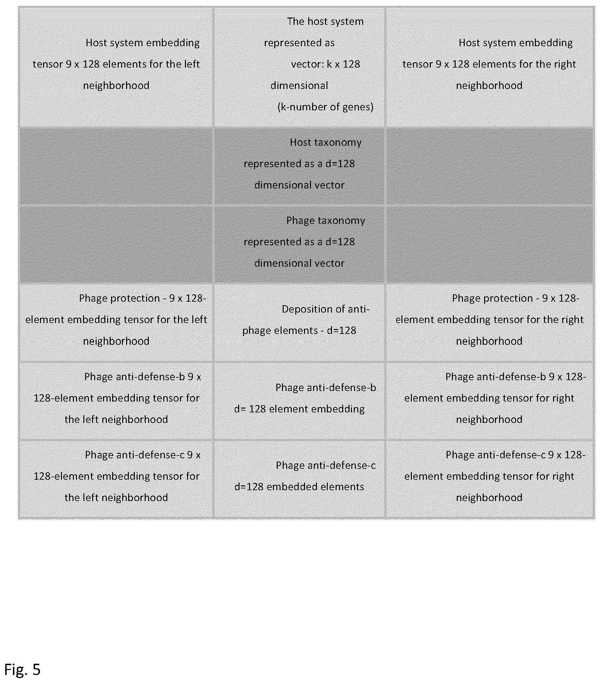

Finally, phage and host pairs were represented as tensors with phage anti-defence genes and phage taxonomy on one side and host taxonomy and host defence genes in that order (navigating vertically from the top of the tensor to the bottom as in Fig. 5). Representations for the phage's anti-defence genes include the upstream and downstream genes in the vicinity of the gene in question. In contrast, representations for the host defence system include the defence genes included in its synteny and the contexts of the upstream and downstream genes (as described in section 2.2.3). The tensors thus defined were then fed into a convolutional neural network (CNN), reconstructing phage-host interactions.

Figs. 4, 5 and 6 show the overall processing scheme and how the tensor for a given phage-host pair is constructed and the architecture of the phage-host interaction model.

Two independent genomic sequences, corresponding to bacteria and phage respectively, together with the protein collections present, are an essential source of data to perform a multivariate analysis to effectively determine the probability of whether there is an infectious process from the phage against the bacterial host or whether the host is able to carry out a defence process.

2.2.1. DNA conversion layer

In the first processing step, the genomes of viruses and their bacterial hosts are subjected to: a. translation of genes into proteins, b. functional annotation of genes. Translation into proteins is performed using Prodigal v2.6.3 [4]. Under annotation - defence systems in bacterial genomes are identified using PADLOC v1.0.1 [5] and DefenseFinder v1.0.9 [6] and subjected to splicing, while genes in phage genomes are identified using multiPhATE2 v2.1 [7].

2.2.2. Intermediate model layer and protein coding

The intermediate models layer included activities related to classifiers and coding methods, necessary for:

a. Identification of relevant proteins on the bacteriophage side. An indicator model implementing this protein identification function is presented in Stage I,
b. Identification of essential proteins on the bacterial side

To effectively identify bacterial defence systems, there is a need to process bacterial genome data in search of defence systems confirmed so far in the scientific literature. As all of these are continuously updated (due to newly confirmed laboratory studies) in the Padloc [5] and DefenseFinder [6] software, it is optimal to use them as part of the systems annotation. Prodigal was used to transform the genome into a proteome. They are then used by Padloc [5] to identify defence systems proteins in the proteome. DefenseFinder [6] uses the proteomes generated by Padloc [5] and tags the proteins in a Padloc [5]-independent manner. The format of the DefenseFinder [6] results is then adapted to what Padloc [5] generates. The two sources of results are combined and duplicates are removed (some cases are labelled the same by both programs). This procedure is applied to any bacterial genome. The result is a list of defence systems (provided as protein by protein), which will then be used to further encode defence islands. Below is the format of the record describing the defence system in question:

```
Protein_name - name of the protein of a given defence system
Gene_type - the gene type of a particular defence system
Defense_system_name - scientific name of the defense system
Bp_range_in_genome - the range of a gene in a given genome sequence (given in
base positions ( bp ))
Protein_sequence - amino acid sequence of the protein
```

Coding of proteins indicated by intermediate models.

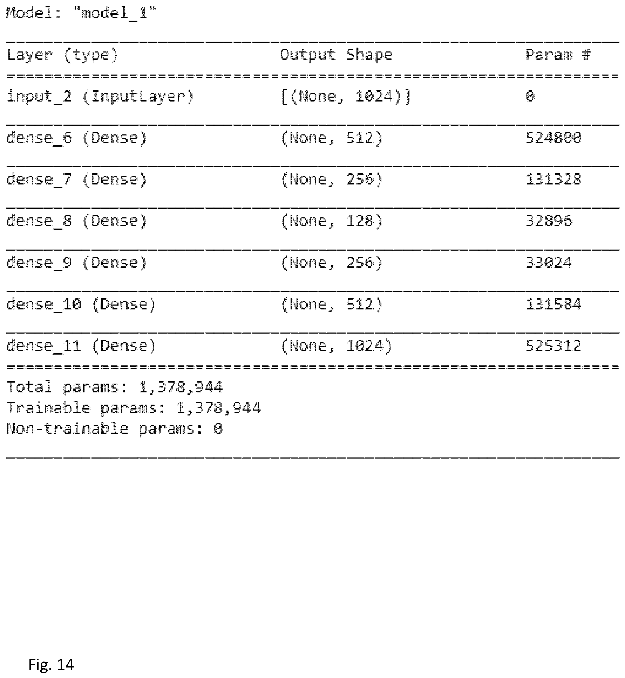

For proteins indicated by the intermediate model on the phage side (aforementioned subsection a) - the proteins are encoded using ProtBert up to dimension d=1024. The dense vectors thus prepared are then subjected to dimension reduction by a dedicated Auto-Encoder with the architecture presented in Fig. 14. It performs the reduction from a vector of dimension d=1024 to a vector of d=128.

The Auto-Encoder consists of:

- input layer with size(number of neurons): 1024

- 3 dense layers of size sequentially: 512, 256, 128 - where 128 is the encoder output A suitable collection saturating the phage-side gene population should be used to train the above Auto-Encoder. In the case of the method described, a collection named as Prokaryotic Virus Orthologous Groups (pVOGs) was used [17].

For proteins that are produced by genes that are part of the defence systems and their contexts (+-n genes) indicated by an intermediate model on the host side (the aforementioned subsection b), coding using ProtBert was applied, but subjected to a learning process: transfer learning. This allows the statistical distribution of learned proteins by ProtBert to be adapted to the distribution of proteins produced by genes present on so-called bacterial host defence islands. This methodology uses a set of observed genes from defence syntenes and their context genes (+-n genes), which are fed through an intermediate layer in (b) on a set of bacterial hosts: the GTDB[18]. The vectors ultimately produced by transfer learning have dimension d=128.

The dense vectors finally produced in both subsections (a and b) are consistent in the sense of dimension (d=128) which is important for the encoding of the input tensor from Fig. 5 and further used as input to the models in Stage II.

### 2.2.3 Context coding

Patterns generated by molecular biology occur both at the level of multigene systems, which appear, for example, as gene syntenes, but also at the level of synergies/antagonisms of the defence systems concerned (pan-immune level). Adjacent genes may share specific physiological processes. In addition, proteins formed by syntenic genes form different types of patterns. For example, the scientific world has observed that the protein domains of individual polygenic patterns (here: defence systems) are similar between co-evolving systems. Therefore, it is important to capture both types of these properties simultaneously (syntenic and proteomic), so that a model reconstructing the manifold topology of a given phenotype can see the patterns of co-evolving phenotypes at multiple levels. In practice, we can observe specific constellation patterns that form clusters in the embedded space, as described above.

The resulting solution allows polygenic systems to be encoded as second-order tensors and as graph structures.

As mentioned above, since neighbouring genes, including those that are part of synteny, can participate in specific physiological processes, and at the same time the proteins produced by each gene may have evolutionarily similar mechanisms of action (especially their attachment domains), simultaneous coding of both types of information is crucial.

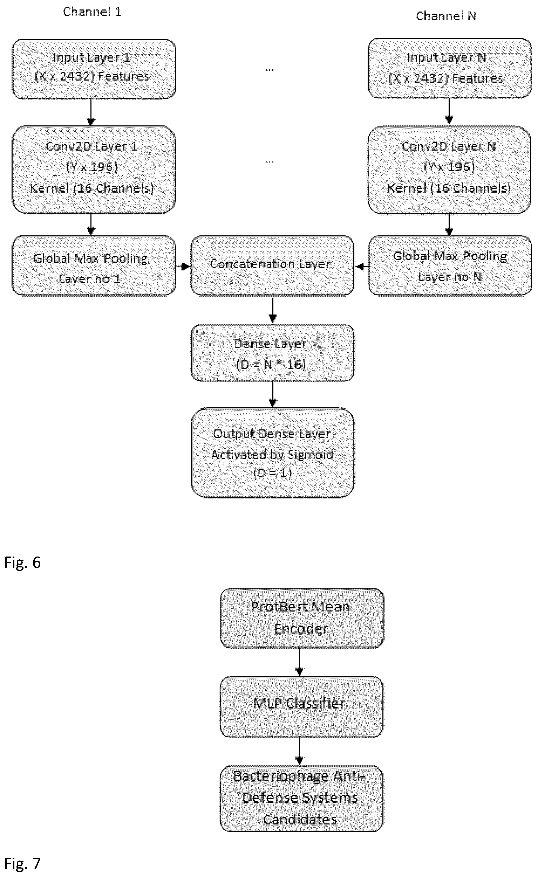

In the first step, for the selected key gene, the method finds its context of neighbouring genes (in this case k=9 downstream genes and k=9 upstream genes). All proteins formed from the key (central) gene and the neighbouring genes are encoded in dense vectors of dimension d = 128, as described in Section 2.2.2. We then combine them, preserving the order, into combined forms into a single tensor, as in Fig. 5. The tensors thus created, reflecting the topology, allow us to capture complex pattern types through the splice operation of the main model (architecture shown in Fig. 6), to which the aforementioned tensor is the input.

### 2.2.4 Taxonomic assignment

Taxonomy refers to a biological classification that refers to the division of a given organism (including phages and bacterial hosts) into groups or taxa (sing, taxon) based on their mutual similarity or evolutionary relatedness.

In order to train the models - a corpus was collected: for phages in number: 12721 and for bacterial hosts: 45353. For both phages and hosts, their classification labels were downloaded from the NCBI database.

For both taxonomic classifiers (individually for: phages and bacterial hosts), a fractal-like graphical representation was used to identify complex patterns through splicing. In particular, the approach: Frequence Chaos Game Representation [14], which we used in this invention, allows the efficient encoding of whole genomes into images (second-order tensors). Each pixel has an individual numerical value that encodes the frequency of occurrence of a given k-mer.

• Determining the taxonomic origin of the bacteriophage genome

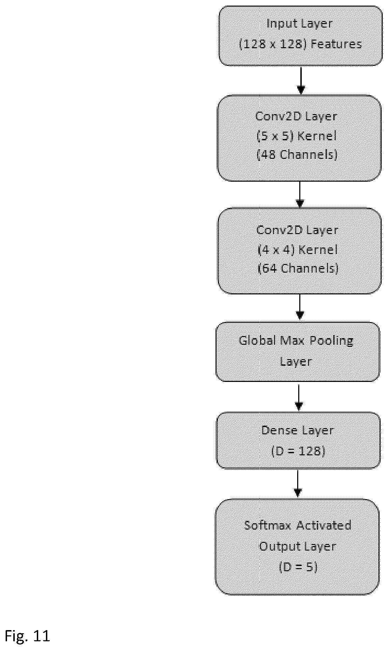

In order to determine the taxonomic affiliation of the bacteriophage, a convolutional neural network model was created using the Tensorflow library with the architecture described in the diagram shown in Fig. 11. This model accepts second-order tensors, prepared according to the FCGR approach described above. Such a model for a single phage genome, encoded to form an FCGR tensor, produces:

    a. probability of belonging to a specific taxonomic group
    b. dense vectors (coming from the penultimate layer of the model - the so-called Dense Layer of the model as in Fig. 11)

In particular, the dense vectors as above are used in the present invention as appended vectors to encode the bulk tensor as in Fig. 5 for the purpose of classifying the Fag-Host interaction as in Fig. 6.

• Determining the taxonomic origin of the bacterial genome:
To determine the taxonomic affiliation of bacteria, a convolutional network model was created using the Tensorflow library. Taxonomic classification is based on the entire bacterial genome. The taxonomic assignment classes used in the classification process were taken from the order level of the official taxonomic system. In practice, a graphical (fractal) representation of the genome is performed using the FCGR method, which aims to systematise the frequency of k-mer subsequences of a given length in a numerical matrix.

Similar to the taxonomic classifier for phages described above - the bacterial taxonomic classification model produces:

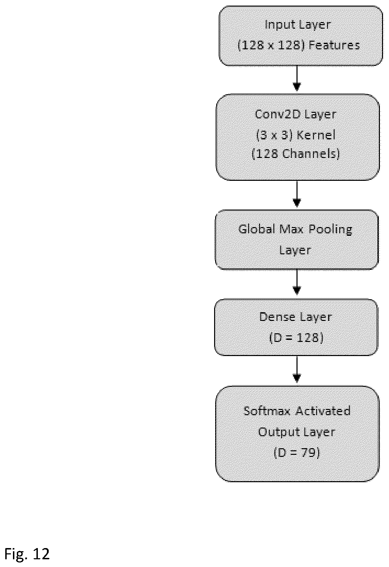

a. probability of belonging to a specific taxonomic group
b. dense vectors (coming from the penultimate layer of the model - the so-called Dense Layer of the model as in Fig. 12)

The created vectors as above dense are used in the present invention as vectors attached to the encoding of the pooled tensor as in Fig. 5 for the purpose of classifying the Fag-Host interaction as in Fig. 6.

2.2.7. Output layer

The results of the method according to the invention are:

a. the value of the probability of infection of the bacterial organism by the designated candidate bacteriophage.
b. during the execution of the method, a 3D visualisation is generated, locating the analysed case in the functional metric space, reproducing the infection-resistance function in comparison with other representations in the form of bacteria-bacteriophage pairs. The location of an object (numerical representation of a bacterium-bacteriophage pair) in the aforementioned metric space is determined by its adopted infection probability value.
c. identification of genes on the phage side that are responsible for inactivating the action of a specific defence system of a given bacterial host (so-called anti-defence gene).
d. indication of the functional linkage of a phage gene identified as in 'c' above to: a specific defence system or gene included in its gene synteny on the bacterial host side. Usually these pairings are confirmed as Protein-Protein Interaction ( i.e. forming a protein complex).

Literature:

[1] ProtBert - ProtTrans: Towards Cracking the Language of Life's Code Through Self-Supervised Learning - Ahmed Elnaggar, Michael Heinzinger, Christian Dallago, Ghalia Rehawi, Yu Wang, Llion Jones, Tom Gibbs, Tamas Feher, Christoph Angerer, Martin Steinegger, Debsindhu Bhowmik, Burkhard Rost, 2021
[2] Hugging Face - https://https://huggingface.co/
[3] Uniref100 - UniRef: comprehensive and non-redundant UniProt reference cluster - Baris E. Suzek, Hongzhan Huang, Peter McGarvey, Raja Mazumder, Cathy H. Wu Author Notes 2007 - https://www.uniprot.org/help/uniref
[4] Prodigal - Prodigal: prokaryotic gene recognition and translation initiation site identification - Doug Hyatt, Gwo-Liang Chen, Philip F LoCascio, Miriam L Land, Frank W Larimer & Loren J Hauser, 2010
[5] PADLOC - Payne, L. J., Todeschini, T. C., Wu, Y., Perry, B. J., Ronson, C. W., Fineran, P. C., Nobrega, F. L., Jackson, S. A. (2021) Identification and classification of antiviral defence systems in bacteria and archaea with PADLOC reveals new system types. Nucleic Acids Research, 49, 10868-10878. doi: https://doi.org/10.1093/nar/gkab883
[6] DefenseFinder - "Systematic and quantitative view of the antiviral arsenal of prokaryotes" Nature Communication, 2022, Tesson F., Hervé A. , Mordret E., Touchon M., d'Humières C., Cury J., Bernheim A.
[7] multiPhATE2 - MultiPhATE2: code for functional annotation and comparison of phage genomes - Carol L Ecale Zhou, Jeffrey Kimbrel, Robert Edwards, Katelyn McNair, Brian A Souza, Stephanie Malfatti, 2021
[8] dbAPIS: a database of anti-prokaryotic immune system genes - Yuchen Yan, Jinfang Zheng, Xinpeng Zhang, Yanbin Yin, 2024
[9] IMGVR - IMG/VR v4: an expanded database of uncultivated virus genomes within a framework of extensive functional, taxonomic, and ecological metadata - Antonio Pedro Camargo, Stephen Nayfach, I-Min A Chen, Krishnaveni Palaniappan, Anna Ratner, Ken Chu, Stephan J Ritter, T B K Reddy, Supratim Mukherjee, Frederik Schulz, Lee Call, Russell Y Neches, Tanja Woyke, Natalia N Ivanova, Emiley A Eloe-Fadrosh, Nikos C Kyrpides, Simon Roux, 2023
[10] Bernheim, A. and Sorek, R., 2020. The pan-immune system of bacteria: antiviral defence as a community resource. Nature reviews microbiology, 18(2), pp.113-119.
[11] Shrikumar, A., Greenside, P. and Kundaje, A., 2017, July. Learning important features through propagating activation differences. In International conference on machine learning (pp. 3145-3153). PMLR.
[12] Lundberg, S.M. and Lee, S.I., 2017. A unified approach to interpreting model predictions. Advances in neural information processing systems, 30.

[13] Yin, Y., Yang, B. and Entwistle, S., 2019. Bioinformatics identification of anti-CRISPR loci by using homology, guilt-by-association, and CRISPR self-targeting spacer approaches. Msystems, 4(5), pp.10-1128.

[14] Löchel, H.F. and Heider, D., 2021. Chaos game representation and its applications in bioinformatics. Computational and structural biotechnology journal, 19, pp.6263-6271.

[15] Srikant, S., Guegler, C.K. and Laub, M.T., 2022. The evolution of a counter-defense mechanism in a virus constrains its host range. Elife, 11, p.e79549.

[16] Yirmiya, E., Leavitt, A., Lu, A., Ragucci, A.E., Avraham, C., Osterman, I., Garb, J., Antine, S.P., Mooney, S.E., Hobbs, S.J. and Kranzusch, P.J., 2024. Phages overcome bacterial immunity via diverse anti-defence proteins. Nature, 625(7994), pp.352-359.

[17] Grazziotin, A.L., Koonin, E.V. and Kristensen, D.M., 2016. Prokaryotic Virus Orthologous Groups (pVOGs): a resource for comparative genomics and protein family annotation. Nucleic acids research, p.gkw975.

[18] Parks, D.H., Chuvochina, M., Rinke, C., Mussig, A.J., Chaumeil, P.A. and Hugenholtz, P., 2022. GTDB: an ongoing census of bacterial and archaeal diversity through a phylogenetically consistent, rank normalized and complete genome-based taxonomy. Nucleic acids research, 50(D1), pp.D785-D794.

## Claims

1. computer-aided method for modelling phage-host interactions using deep-architecture neural networks and machine learning, according to the invention, is **characterised in that** it is carried out in the following steps:

   Stage I - application of the directional model:

   1) identifying known defence systems on the host side - a group of at least 91 candidate defence systems;
   2) Identifying known phage-side anti-defence systems using an indicator model - a group of at least 20 candidate anti-defence systems - cases with a probability of belonging to a class above 0.9 are selected;
   3) The presence of individual protein groups within the virus and host is identified in the model input matrix by 3 values in the range (0 - 1);
   4) at the intersection of the two occurring defence and anti-defence systems, the value 1.0 appears, with the positions of the occurrence of a single system represented as 0.5, with the remaining fields filled in by the value 0.0. This type of representation takes into account all combinations of defence - anti-defence systems without pre-directing the model analysis to the selected group of proteins;
   5) The data representation described is used to build a classification model using two-dimensional convolution layers, with the aim of the network being to assess the probability of virus-host interaction based on the co-occurrence matrix of bacterial and viral systems;
   6) The convolutional network in the next stage is analysed using Shapley values, with the highest values in the input matrix indicating a group of defence-anti-defence systems that are of particular importance in the classifier's decision-making process, with the designated systems being used in stage II of the construction of classification models targeting detailed information about protein systems;

   Stage II - application of models of a specific group of defence and anti-defence systems:

   1) identifying the host-side defence systems identified by Stage I;
   2) identification of the bacteriophage-side systems (reference genes/proteins) indicated by step I;
   3) each of the above-identified genes producing reference (bacteriophage/host) proteins, are encoded in a form that expresses both:

   a. protein properties for a reference set of genes (gene synteny),
   b. properties of proteins produced by the context of neighbouring genes relative to the reference gene synteny,
   c. coding is in the form of dense vectors and is carried out in the following way:
   for a selected reference gene synteny, the context of the neighbouring genes is established (n genes upstream and n genes downstream of the gene synteny), whereby all proteins formed from sequences of key genes (central, belonging to the synteny of the defense system in question) and neighboring genes are encoded with the help of dense vectors of dimension d = 128, these proteins are then combined, preserving the order of appearance of the genes to which they refer, into a combined form in a single tensor. The tensors thus created, reflecting the topology of the genes, further allow complex types of syntenic patterns to be established through a Convolutional Neural Network (CNN) master model operation;

4) Coding phage taxonomy to capture pan-immune patterns;

5) Encoding host taxonomy to capture pan-immune patterns;

6) concatenation of all the above encoded dense vectors in the form of an input tensor ;

7) Training an AI/ML (Convolutional Neural Network) model of phage-host interaction using a dataset of phage-host pairs;

8) for a given input tensor of a phage-host pair, the identification of which of its features are most relevant to the decision-making process (Shapley value) by the neural network for: infection/resistance phenotypes, with these features being mapped to the proteins encoded in the phage-host tensor.

| | Bacteria system no 1 | Bacteria system no 2 | Bacteria system no 3 | Bacteria system no 4 | Bacteria system no 5 | Bacteria system no 6 | Bacteria system no 7 | Bacteria system no 8 | ... |
|---|---|---|---|---|---|---|---|---|---|
| Phage system no 1 | | | | | | | | | |
| Phage system no 2 | | | | | | | | | |
| Phage system no 3 | | | | | | | | | |
| Phage system no 4 | | | | | | | | | |
| Phage system no 5 | | | | | | | | | |
| ... | | | | | | | | | |

Fig. 1

Fig. 2

Fig. 3

Fig. 4

| Left context proteins | Central Protein | Proteins in the right context |
|---|---|---|

| Host system embedding tensor 9 x 128 elements for the left neighborhood | The host system represented as vector: k x 128 dimensional (k-number of genes) | Host system embedding tensor 9 x 128 elements for the right neighborhood |
|---|---|---|
| | Host taxonomy represented as a d=128 dimensional vector | |
| | Phage taxonomy represented as a d=128 dimensional vector | |
| Phage protection - 9 x 128-element embedding tensor for the left neighborhood | Deposition of anti-phage elements - d=128 | Phage protection - 9 x 128-element embedding tensor for the right neighborhood |
| Phage anti-defense-b 9 x 128-element embedding tensor for the left neighborhood | Phage anti-defense-b d= 128 element embedding | Phage anti-defense-b 9 x 128-element embedding tensor for right neighborhood |
| Phage anti-defense-c 9 x 128-element embedding tensor for the left neighborhood | Phage anti-defense-c d=128 embedded elements | Phage anti-defense-c 9 x 128-element embedding tensor for right neighborhood |

Fig. 5

Channel 1 ... Channel N

Input Layer 1
(X x 2432) Features

Conv2D Layer 1
(Y x 196)
Kernel (16 Channels)

Global Max Pooling
Layer no 1

...

Input Layer N
(X x 2432) Features

Conv2D Layer N
(Y x 196)
Kernel (16 Channels)

Global Max Pooling
Layer no N

Concatenation Layer

Dense Layer
(D = N * 16)

Output Dense Layer
Activated by Sigmoid
(D = 1)

Fig. 6

ProtBert Mean
Encoder

MLP Classifier

Bacteriophage Anti-
Defense Systems
Candidates

Fig. 7

Fig. 8

Left oriented
protein context

Defense System
Synteny

Right oriented
protein context

Fig. 9

a)  Virus

b) Host

Fig. 10

Fig. 11

Fig. 12

Fig. 13

```
Model: "model_1"

_____
Layer (type)                 Output Shape              Param #
=================================================================
input_2 (InputLayer)         [(None, 1024)]            0

_____
dense_6 (Dense)              (None, 512)               524800

_____
dense_7 (Dense)              (None, 256)               131328

_____
dense_8 (Dense)              (None, 128)               32896

_____
dense_9 (Dense)              (None, 256)               33024

_____
dense_10 (Dense)             (None, 512)               131584

_____
dense_11 (Dense)             (None, 1024)              525312
=================================================================
Total params: 1,378,944
Trainable params: 1,378,944
Non-trainable params: 0

_____
```

Fig. 14

Europäisches
Patentamt

European
Patent Office

Office européen
des brevets

# EUROPEAN SEARCH REPORT

**Application Number**

EP 24 17 7290

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LOOD CÉDRIC ET AL: "Digital phagograms: predicting phage infectivity through a multilayer machine learning approach", CURRENT OPINION IN VIROLOGY, vol. 52, 21 December 2021 (2021-12-21), pages 174-181, XP093229250, United Kingdom ISSN: 1879-6257, DOI: 10.1016/j.coviro.2021.12.004 * Abstract; sections "Features available to learn models", "Exploring phage-bacteria interactions using pangenomes and bags of genes", and "A layered approach to interaction modelling"; figures 1,2; table 1 * | 1 | INV. G16B5/00 G16B20/00 G16B40/20 |
| X | Gaborieau Baptiste ET AL: "Predicting phage-bacteria interactions at the strain level from genomes", bioRxiv, 22 November 2023 (2023-11-22), XP093229248, DOI: 10.1101/2023.11.22.567924 Retrieved from the Internet: URL:https://www.biorxiv.org/content/10.1101/2023.11.22.567924v1.full.pdf [retrieved on 2024-11-28] * Abstract; Results (sections "Defense systems are not required to predict ..." on page 14 and "Adsorption factors are sufficient to..." on page 16); Methods (sections "Detection of genomic traits from the genomes of phages and bacteria" and "Training algorithms to predict phage-bacteria interactions"); figure 6 * | 1 | |

| TECHNICAL FIELDS SEARCHED (IPC) |
|---|
| G16B |

-----

-/--

The present search report has been drawn up for all claims

| Place of search | Date of completion of the search | Examiner |
|---|---|---|
| Munich | 29 November 2024 | Hendrikse, Natalie |

EPO FORM 1503 03.82 (P04C01)

**page 1 of 3**

**EUROPEAN SEARCH REPORT**

Europäisches
Patentamt
European
Patent Office
Office européen
des brevets

Application Number

EP 24 17 7290

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WANG YUE ET AL: "An Effective Model for Predicting Phage-Host Interactions Via Graph Embedding Representation Learning With Multi-Head Attention Mechanism", IEEE JOURNAL OF BIOMEDICAL AND HEALTH INFORMATICS, IEEE, PISCATAWAY, NJ, USA, vol. 27, no. 6, 24 March 2023 (2023-03-24), pages 3061-3071, XP011942229, ISSN: 2168-2194, DOI: 10.1109/JBHI.2023.3261319 [retrieved on 2023-03-27] * the whole document * ----- | 1 | |
| X | PAN JIE ET AL: "GSPHI: A novel deep learning model for predicting phage-host interactions via multiple biological information", COMPUTATIONAL AND STRUCTURAL BIOTECHNOLOGY JOURNAL, [Online] vol. 21, 16 June 2023 (2023-06-16), pages 3404-3413, XP093228911, Sweden ISSN: 2001-0370, DOI: 10.1016/j.csbj.2023.06.014 Retrieved from the Internet: URL:https://pmc.ncbi.nlm.nih.gov/articles/PMC10314231/pdf/main.pdf> [retrieved on 2024-11-28] * the whole document * ----- | 1 | TECHNICAL FIELDS SEARCHED (IPC) |

-/--

The present search report has been drawn up for all claims

| Place of search | Date of completion of the search | Examiner |
|---|---|---|
| Munich | 29 November 2024 | Hendrikse, Natalie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 3

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | NIE WANCHUN ET AL: "Advances in phage-host interaction prediction: in silico method enhances the development of phage therapies", BRIEFINGS IN BIOINFORMATICS, vol. 25, no. 3, 28 March 2024 (2024-03-28) , XP093228918, GB ISSN: 1467-5463, DOI: 10.1093/bib/bbae117 * the whole document * ----- | 1 | |

TECHNICAL FIELDS SEARCHED (IPC)

The present search report has been drawn up for all claims

| Place of search | Date of completion of the search | Examiner |
|---|---|---|
| Munich | 29 November 2024 | Hendrikse, Natalie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................

& : member of the same patent family, corresponding document

EPO FORM 1503 03.82 (P04C01)

page 3 of 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **AHMED ELNAGGAR ; MICHAEL HEINZINGER ; CHRISTIAN DALLAGO ; GHALIA REHAWI ; YU WANG ; LLION JONES ; TOM GIBBS ; TAMAS FEHER ; CHRISTOPH ANGERER ; MARTIN STEINEGGER**. *ProtTrans: Towards Cracking the Language of Life's Code Through Self-Supervised Learning*, 2021
- **BARIS E. SUZEK ; HONGZHAN HUANG ; PETER MCGARVEY ; RAJA MAZUMDER ; CATHY H. WU**. *UniRef: comprehensive and non-redundant UniProt reference cluster*, 2007, https://www.uniprot.org/help/uniref
- **DOUG HYATT ; GWO-LIANG CHEN ; PHILIP F LOCASCIO ; MIRIAM L LAND ; FRANK W LARIMER ; LOREN J HAUSER**. *Prodigal: prokaryotic gene recognition and translation initiation site identification*, 2010
- **PAYNE, L. J. ; TODESCHINI, T. C. ; WU, Y. ; PERRY, B. J. ; RONSON, C. W. ; FINERAN, P. C. ; NOBREGA, F. L. ; JACKSON, S. A.** Identification and classification of antiviral defence systems in bacteria and archaea with PADLOC reveals new system types. *Nucleic Acids Research*, 2021, vol. 49, 10868-10878, https://doi.org/10.1093/nar/gkab883
- **TESSON F. ; HERVÉ A. ; MORDRET E. ; TOUCHON M. ; D'HUMIÈRES C. ; CURY J. ; BERNHEIM A.** Systematic and quantitative view of the antiviral arsenal of prokaryotes. *Nature Communication*, 2022
- **CAROL L ECALE ZHOU ; JEFFREY KIMBREL ; ROBERT EDWARDS ; KATELYN MCNAIR ; BRIAN A SOUZA ; STEPHANIE MALFATTI**. *MultiPhATE2: code for functional annotation and comparison of phage genomes*, 2021
- **YUCHEN YAN ; JINFANG ZHENG ; XINPENG ZHANG ; YANBIN YIN**. *dbAPIS: a database of anti-prokaryotic immune system genes*, 2024
- **ANTONIO PEDRO CAMARGO ; STEPHEN NAYFACH ; I-MIN A CHEN ; KRISHNAVENI PALANIAPPAN ; ANNA RATNER ; KEN CHU ; STEPHAN J RITTER ; T B K REDDY ; SUPRATIM MUKHERJEE ; FREDERIK SCHULZ**. *IMG/VR v4: an expanded database of uncultivated virus genomes within a framework of extensive functional, taxonomic, and ecological metadata*, 2023
- **BERNHEIM, A. ; SOREK, R.** The pan-immune system of bacteria: antiviral defence as a community resource. *Nature reviews microbiology*, 2020, vol. 18 (2), 113-119
- **SHRIKUMAR, A. ; GREENSIDE, P. ; KUNDAJE, A.** Learning important features through propagating activation differences. *International conference on machine learning*, July 2017, 3145-3153
- **LUNDBERG, S.M. ; LEE, S.I.** A unified approach to interpreting model predictions. *Advances in neural information processing systems*, 2017, vol. 30
- **YIN, Y. ; YANG, B. ; ENTWISTLE, S.** Bioinformatics identification of anti-CRISPR loci by using homology, guilt-by-association, and CRISPR self-targeting spacer approaches. *Msystems*, 2019, vol. 4 (5), 10-1128
- **LÖCHEL, H.F. ; HEIDER, D.** Chaos game representation and its applications in bioinformatics. *Computational and structural biotechnology journal*, 2021, vol. 19, 6263-6271
- **SRIKANT, S. ; GUEGLER, C.K. ; LAUB, M.T.** The evolution of a counter-defense mechanism in a virus constrains its host range. *Elife*, 2022, vol. 11, e79549
- **YIRMIYA, E. ; LEAVITT, A. ; LU, A. ; RAGUCCI, A.E. ; AVRAHAM, C. ; OSTERMAN, I. ; GARB, J. ; ANTINE, S.P. ; MOONEY, S.E. ; HOBBS, S.J.** Phages overcome bacterial immunity via diverse anti-defence proteins. *Nature*, 2024, vol. 625 (7994), 352-359
- **GRAZZIOTIN, A.L. ; KOONIN, E.V. ; KRISTENSEN, D.M.** Prokaryotic Virus Orthologous Groups (pVOGs): a resource for comparative genomics and protein family annotation. *Nucleic acids research*, 2016
- **PARKS, D.H. ; CHUVOCHINA, M. ; RINKE, C. ; MUSSIG, A.J. ; CHAUMEIL, P.A. ; HUGENHOLTZ, P.** GTDB: an ongoing census of bacterial and archaeal diversity through a phylogenetically consistent, rank normalized and complete genome-based taxonomy. *Nucleic acids research*, 2022, vol. 50 (D1), D785-D794